(19) Europäisches Patentamt
European Patent Office
Office européen des brevets

(11) EP 2 987 416 B1

(12) # EUROPEAN PATENT SPECIFICATION

(45) Date of publication and mention of the grant of the patent:
**25.04.2018 Bulletin 2018/17**

(21) Application number: **14763921.5**

(22) Date of filing: **13.03.2014**

(51) Int Cl.:
***A23L 27/10*** *(2016.01)* ***A23L 2/44*** *(2006.01)*
***A23L 2/56*** *(2006.01)* ***A23L 3/3472*** *(2006.01)*
***A23L 27/00*** *(2016.01)* ***A23C 9/152*** *(2006.01)*
***A23C 13/10*** *(2006.01)* ***A23G 9/42*** *(2006.01)*
***A23L 9/10*** *(2016.01)* ***A23C 9/133*** *(2006.01)*
***A23L 2/02*** *(2006.01)* ***A23G 3/48*** *(2006.01)*
***A61Q 13/00*** *(2006.01)* ***A61K 8/97*** *(2017.01)*

(86) International application number:
**PCT/JP2014/056805**

(87) International publication number:
**WO 2014/142287 (18.09.2014 Gazette 2014/38)**

(54) **FLAVOR DETERIORATION INHIBITOR**

DUFTSTOFFZERSETZUNGSHEMMER

INHIBITEUR DE DÉTÉRIORATION DE SAVEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.03.2013 JP 2013052133**

(43) Date of publication of application:
**24.02.2016 Bulletin 2016/08**

(73) Proprietor: **Takasago International Corporation**
**Tokyo 144-8721 (JP)**

(72) Inventors:
- **OHMIYA Tadamasa**
  **Hiratsuka-shi**
  **Kanagawa 254-0073 (JP)**
- **HIRAMOTO Tadahiro**
  **Hiratsuka-shi**
  **Kanagawa 254-0073 (JP)**
- **GONDA Yoshiharu**
  **Hiratsuka-shi**
  **Kanagawa 254-0073 (JP)**

(74) Representative: **Takeuchi, Maya et al**
**Fédit-Loriot**
**38, avenue Hoche**
**75008 Paris (FR)**

(56) References cited:
**EP-A1- 2 172 537** **JP-A- S61 112 024**
**JP-A- S61 112 024** **JP-A- 2001 136 931**
**JP-A- 2001 136 931** **JP-A- 2003 033 164**
**JP-A- 2008 285 637** **JP-A- 2012 153 671**
**JP-A- 2012 153 671** **JP-A- 2012 231 796**
**US-B1- 6 265 011**

- **JIMENEZ-ALVAREZ D ET AL: "Antioxidant activity of oregano, parsley, and olive mill wastewaters in bulk oils and oil-in-water emulsions enriched in fish oil", JOURNAL OF AGRICULTURAL AND FOOD CHEMISTRY, AMERICAN CHEMICAL SOCIETY, US, vol. 56, no. 16, 27 August 2008 (2008-08-27), pages 7151-7159, XP002718019, ISSN: 0021-8561, DOI: 10.1021/JF801154R [retrieved on 2008-07-18]**
- **ZHANG ET AL: "Evaluation of antioxidant activity of parsley (Petroselinum crispum) essential oil and identification of its antioxidant constituents", FOOD RESEARCH INTERNATIONAL, ELSEVIER APPLIED SCIENCE, BARKING, GB, vol. 39, no. 8, 1 October 2006 (2006-10-01), pages 833-839, XP005591409, ISSN: 0963-9969, DOI: 10.1016/J.FOODRES.2006.03.007**
- **SHAN B ET AL.: 'Antioxidant Capacity of 26 Spice Extracts and Characterization of Their Phenolic Constituents' J AGRIC FOOD CHEM. vol. 53, 2005, pages 7749 - 7759, XP055279480**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **WONG PYY: 'Studies on the dual antioxidant and antibacterial properties of parsley (Petroselinum crispum) and cilantro (Coriandrum sativum) extracts' FOOD CHEMISTRY vol. 97, 2006, pages 505 - 515, XP027989299**

## Description

Technical Field

The present invention relates to a flavor deterioration inhibitor derived from a specific natural product, which can be widely applied to foods or drinks containing a flavor component and also applied to flavors, and a method for inhibiting flavor deterioration.

Background Art

Various products such as foods or drinks and oral hygienic agent have a characteristic aroma, savor or taste owing to the flavor inherent in the material or the flavor generated during production. In particular, in the foods or drinks, the flavor is one of the factors for good taste and also plays an important role in inducing a desire for the food. However, such a flavor component is relatively unstable and therefore, it is known that the flavor is gradually deteriorated by the effect of light, heat, oxygen, etc. in the process of producing, distributing or storing merchandise or during shelving. Above all, in the food and drink field, with the recent popularization of a drink warmed by a hot vending machine or popularization of a drink in a transparent container such as PET bottle, a flavor deterioration phenomenon due to heat or light or a flavor deterioration phenomenon caused during storage or shelving of merchandise arises as a problem, and an inhibitor or method for inhibiting such flavor deterioration has been demanded.

In general, the flavor of a food or drink having a citrus flavor is known to be readily deteriorated particularly by the effect of light, heat, oxygen, etc. In some cases, the fresh flavor peculiar to the original citrus flavor is deteriorated, leading to loss of the flavor of a just-made product or development of an off-flavor. As one of causes thereof, the flavor deterioration is considered to occur because, out of citrus flavor components, citral known as a key component of a lemon flavor, etc. undergoes a cyclization reaction due to light or heat and is transformed to a substance such as photocitral.

Likewise, the flavor of a milk-containing food or drink is also known to be readily deteriorated particularly by the effect of light, heat, oxygen, etc. and as one of causes thereof, it is said that there is a case where an active oxygen is produced due to light by a photosensitizer reaction involving riboflavin in milk, and the milkfat or protein is oxidized by the active oxygen to produce an odor component with a low threshold value, thereby significantly reducing the commercial value.

Among others, in the food and drink field, the selling shelf is set to a very bright condition so as to impart an attractive appeal of merchandise to consumers or deliver a feeling of cleanliness. Furthermore, in order to allow the consumer to confirm the commercial value by allowing the consumer to check the contents of merchandise, offer a sense of safety or help in the decision at the time of purchase of merchandise, a food or drink enclosed in a light-transmitting container such as recent PET bottle, plastic container or transparent bag is wide-spreading. In addition, the selling style is subjected to environmental change, and a selling style of displaying merchandise under a fluorescent lamp for a long period of time in a convenience store, etc. becomes common. Accordingly, it is required to early develop a method capable of significantly inhibiting a flavor deterioration phenomenon due to light or heat or a flavor deterioration phenomenon due to fluorescent lamp irradiation occurring during shelving of merchandise.

Under these backgrounds, various methods, for example, addition of various antioxidants or light deterioration inhibitors such as ascorbic acid, have been heretofore proposed as the method for inhibiting flavor deterioration. Furthermore, it has been proposed that, for example, $\alpha$-glucosylrutin has a flavor deterioration inhibiting effect (see, Patent Document 1), deterioration is inhibited by incorporating a coumarin derivative (see, Patent Document 2), or a tea extract is used in combination and blended with an emulsion containing tocopherol and ferulic acid (Patent Document 3).

On the other hand, the physiological activity of parsley (Apiaceae, scientific name: Petroselinum crispum) is attracting attention, and flavonoid in the parsley leaf extract has been reported to have an antioxidation activity in blood (Non-Patent Document 1). In a document, the parsley leaf extract has been reported to contain an antioxidatively acting component (Non-Patent Document 2). JP2001-136931A discloses a flavor deterioration inhibitor comprising coumarin derivatives.

Prior Art Documents

Patent Documents

Patent Document 1: JP-A-03-27293
Patent Document 2: JP-A-2001-136931

Patent Document 3: JP-A-2004-166631

Non-Patent Documents

Non-Patent Document 1: British Journal of Nutrition, Vol. 81, No. 6 pp. 447-455 (1999)
Non-Patent Document 2: BIN SHAN, YIZHONG Z. CAI, MEI SUN, HAROLD CORKE; JOURNAL OF AGRICALTURAL AND FOOD CHEMISTRY, 2005, 53, 7749-7759

Summary of the Invention

Problems That the Invention is to Solve

With respect to Patent Documents 1 and 2, the natural product-derived deterioration inhibitor in conventional techniques is effective in inhibiting flavor deterioration to a certain extent but lacks in practicability and still fails to exert satisfactory effects, because the deterioration inhibitor needs to be used in a certain large amount for bringing about the flavor deterioration inhibiting effect and in turn, the taste or smell possessed by the flavor deterioration inhibitor itself adversely affects the taste or aroma of a food or drink.

In addition, it has not been conventionally known that the parsley leaf extract has a flavor deterioration inhibiting action. Furthermore, as common knowledge in the food industry, the antioxidative activity and the flavor deterioration inhibiting action are not properties that are univocally associated with each other.

An object of the present invention is to provide a flavor deterioration inhibitor capable of significantly suppressing a flavor deterioration inhibiting phenomenon based on factors such as light. Specifically, an object of the present invention is to provide a flavor deterioration inhibitor that is highly safe and can be suitably used for foods or drinks. Another object of the present invention is to provide a flavor deterioration inhibitor effectively acting on a flavor deterioration inhibiting phenomenon occurring particularly when affected by light out of the factors above. Means for Solving the Problems

The present inventors have made intensive studies on the flavor deterioration inhibition activity of a wide variety of natural product-derived components by focusing on plants so as to attain the objects above, and as a result, it has been found that when a solvent extract of parsley leaves is used, a food or the like can be inhibited remarkably from flavor deterioration due to light and further from flavor deterioration due to heat, oxygen, etc., for a long period of time. Furthermore, it has been confirmed that the excellent flavor deterioration inhibiting effect is not dependent on each of various flavonoids contained in the parsley leaf extract but is a unique effect of the extract itself containing those flavonoids and other trace components in combination. The present invention has been accomplished based on these findings. The invention is best described by the claims. That is, the present invention relates to the following flavor deterioration inhibitor use and the method for inhibiting flavor deterioration.

1. Use of a solvent extract of a parsley leaf as a flavor deterioration inhibitor for inhibiting flavor deterioration due to light.
2. The use according 1, wherein the solvent extract is obtained by extraction with water, an organic solvent or a mixture thereof.
3. A method for inhibiting flavor deterioration of a food or drink due to light, comprising adding a flavor deterioration inhibitor in an amount of from 1 ppb by mass to 500 ppm by mass in terms of solid weight to a food or drink, the flavor deterioration inhibitor comprising a solvent extract of a parsley leaf.
4. The method for inhibiting flavor deterioration of a food or drink according to 3, wherein the solvent extract is obtained by extraction with water, an organic solvent or a mixture thereof.
5.A method for inhibiting flavor deterioration of a flavor due to light comprising adding a flavor deterioration inhibitor in an amount of from 1 ppm by mass to 50 mass % in terms of solid weight to a flavor, the flavor deterioration inhibitor comprising a solvent extract of a parsley leaf.
6. The method for inhibiting flavor deterioration of a flavor according to 5, wherein the solvent extract is obtained by extraction with water, an organic solvent or a mixture thereof.

Advantageous Effects of the Invention

The flavor deterioration inhibitor of the present invention effectively acts on the flavor deterioration inhibiting phenomenon. Among others, a citrus-bsed food or drink, including citral or a citral-containing product, and a milk-containing food or drink is effectively prevented from the progress of flavor deterioration that gradually proceeds in respective stages of production, distribution, storage and shelving, and allowed to maintain a feeling of freshness originally

possessed by the product, whereby the product quality can be maintained inexpensively and stably for a long period of time.

Brief Description of the Drawings

[FIG. 1] FIG. 1 is a graph showing the relationship of various additives and the citral residual ratio.

[FIG. 2] FIG. 2 is a graph showing the relationship of the added amount of the flavor deterioration inhibitor of the present invention and the citral residual ratio.

[FIG. 3] FIG. 3 is a graph showing the relationship of the added amount of the flavor deterioration inhibitor of the present invention and the production ratio of a citral-derived light-deterioration product.

[FIG. 4] FIG. 4 is a graph showing the results of a sensory evaluation of milk to which the flavor deterioration inhibitor of the present invention or other additives has/have been added

[FIG. 5] FIG. 5 is a graph showing the results of a sensory evaluation of a soft ice-cream mix to which the flavor deterioration inhibitor of the present invention or other additives has/have been added

[FIG. 6] FIG. 6 is a graph showing the results of a sensory evaluation of a milk pudding to which the flavor deterioration inhibitor of the present invention or other additives has/have been added

[FIG. 7] FIG. 7 is a graph showing the results of a sensory evaluation of a whipped cream to which the flavor deterioration inhibitor of the present invention or other additives has/have been added.

[FIG. 8] FIG. 8 is a graph showing the results of a sensory evaluation of a yogurt to which the flavor deterioration inhibitor of the present invention or other additives has/have been added.

[FIG. 9] FIG. 9 is an example of the method for producing the flavor deterioration inhibitor of the present invention.

Mode for Carrying Out the Invention

The present invention is described in more detail below. In FIG. 9, an example of the method for producing the flavor deterioration inhibitor of the present invention is depicted.

In the description of the present invention, "mass%", "ppm by mass" and "parts by mass" have the same meanings as "wt%", "ppm by weight" and "parts by weight", respectively.

Furthermore, in the description of the present invention, unless otherwise indicated, ppb, ppm and % means ppb by mass, ppm by mass and mass%, respectively.

(1) Raw Material

Parsley (scientific name: Petroselinum crispum) as the plant for use in the present invention is a biennial in the family Apiaceae native to the Mediterranean region and is a herb commonly used also in Japan as a spice or a relish. The portion to be used is a part including a leaf. The parsley leaf to be used for extraction may be either a just-picked fresh leaf or a leaf after drying it but is preferably a dried leaf.

(2) Extraction Treatment

(a) Solvent

The solvent to be used for the extraction treatment is water or a polar organic solvent, and the organic solvent may be a hydrate.

Examples of the polar organic solvent include an alcohol, acetone, etc. Among these, in view of safety to human body and handling property, water and an aliphatic alcohol having a carbon number of 2 to 4, such as ethanol, propanol and butanol, are preferred.

The amount of the solvent to be used for extraction may be arbitrarily selected but in general, the solvent is used in an amount of 2 to 100 parts by weight per 1 part by weight of the raw material above.

Here, a defatting or cleaning treatment with a non-polar organic solvent such as hexane or a polar organic solvent such as ethyl acetate and ether may be previously performed as a pretreatment for extraction so as to prevent extraction of excess lipid in the subsequent extraction treatment or deodorize and purify the unique flavor or odor of the material. In addition, for the purpose of deodorization, a steam distillation treatment may also be applied before extraction.

(b) Extraction Treatment Method

As the extraction treatment method, various methods can be employed according to the kind, amount and the like of the solvent. For example, the raw material may be added to a solvent and extracted by a dipping method or a heat-refluxing method. In order to increase the extraction efficiency, the raw material may also be pulverized. In the case of a dipping method, the treatment may be performed under any of heating condition, room temperature condition and cooling condition. The extraction temperature may be arbitrarily determined and is not particularly limited, but the suitable temperature is preferably from 0°C to 100°C, more preferably from 50°C to 100°C. In addition, the suitable extraction time is preferably from 1 hour to 72 hours, more preferably from 2 hours to 7 hours.

Subsequently, solid matters insoluble in the solvent are removed to obtain an extraction liquid. As the method for removing solid matters, various solid-liquid separation techniques such as centrifugal separation, filtration and compression may be used.

The obtained extraction liquid may be directly used as a flavor deterioration inhibitor but may be used, for example, after appropriately diluting it with a liquid such as water, ethanol, glycerin and propylene glycol. An excipient such as dextrin may also be added. In addition, the resulting liquid may be further concentrated for use as a paste extract. The paste may be further dried for use as a powder. The method for powder formation is not particularly limited and, for example, hot-air drying, freeze drying, spray drying, drum drying, foam-mat drying, fluidized bed drying or the like can be used.

Furthermore, an extract by supercritical extraction or fractionated or treated for deodorization may also be used.

(c) Purification

The extract obtained by the above-described method may be directly incorporated as a flavor deterioration inhibitor but may be further subjected to a purification treatment such as decolorization or deodorization. In the purification treatment, activated carbon, silicon dioxide, a synthetic resin adsorbent, etc. can be used. As the synthetic resin adsorbent for purification, for example, an adsorbent composed of an aromatic resin, an acrylic resin, an acrylonitrile-based resin, etc. can be used. Such a synthetic resin adsorbent is commercially available, and examples thereof include DIAION HP-20, HP21 and SEPABEADS SP70 (all aromatic resin, produced by Mitsubishi Chemical Corporation) ("DIAION" and "SEPABEADS" are registered trademarks, hereinafter the same)"; SEPABEADS SP825, SP850 and SP700 (all aromatic high surface area-type resin, produced by Mitsubishi Chemical Corporation); SEPABEADS SP207 (aromatic modified resin, produced by Mitsubishi Chemical Corporation); DIAION HP20SS, SEPABEADS SP20SS and SP207SS (all aromatic small particle size-type resin, produced by Mitsubishi Chemical Corporation); AMBERLITE XAD2, XAD4, FPX66, XAD1180, XAD1180N and XAD2000 (all styrene-based resin, produced by Organo Corporation) ("AMBERLITE" is a registered trademark, hereinafter the same); DIAION HP2MG (acrylic resin, produced by Mitsubishi Chemical Corporation); AMBERLITE XAD7HP (acrylic resin, produced by Organo Corporation); and SEPHADEX LH20 (crosslinked dextran derivative, produced by GE Healthcare Bioscience) ("SEPHADEX" is a registered trademark).

(d) Concentration

The extract obtained by the above-described method may be concentrated before use. As the concentration method, the concentration may be done by a generally conventional method. For example, the solvent may be recovered using an evaporator or in the case of using a synthetic adsorbent resin, it is also possible to concentrate the extract by using an eluent in a small amount relative to the amount of the solution loaded.

(3) Preparation of Flavor Deterioration Inhibitor

The flavor deterioration inhibitor of the present invention may be sufficient if it contains the above-described parsley leaf extract (an extraction liquid itself, a concentrate, a dried product or a purified product), and the flavor deterioration inhibitor may be composed of only such an extract but may contain a diluent, a carrier or other additives, as a component other than the extract.

The diluent or carrier is not particularly limited as long as it does not inhibit the effects of the present invention, and examples thereof include sugars such as sucrose, glucose, dextrin, starches, cyclodextrin, trehalose, lactose, maltose, palatinose, xylose, starch syrup and liquid sugar; alcohols such as ethanol, propylene glycol and glycerin; sugar alcohols such as reduced starch syrup, reduced maltose syrup, sorbitol, lactitol, palatinit, mannitol, xylitol, erythritol and maltitol; polysaccharides such as gum arabic, xanthan gum, carrageenan, guar gum and gellan gum; and water. Examples of the additive include an antioxidant, an auxiliary agent such as chelating agent, a flavor or fragrance, a spice extract, an edible plant extract, a fruit extract, a low-boiling-point fraction of cold pressed oil, an antiseptic, etc.

As other additives, the flavor deterioration inhibitor may be used in combination with a conventional antioxidant

(for example, BHT, BHA, α-tocopherol, and vitamin C), preservative (for example, sorbic acid, benzoic aid, and soft roe protein extract), shelf-life improver (for example, sodium acetate, glycine, glycerin fatty acid ester, lysozyme, and xanthone), etc.

From the standpoint of convenience and the like in use, in the case of preparing the flavor deterioration inhibitor by using such a diluent, carrier or additive, the preparation is preferably adjusted such that the parsley leaf extract is contained in a ratio of 1 ppm to 50% in terms of solid matter in 100% of the flavor deterioration inhibitor. In the case where the target product is a food or drink, the preparation is preferably adjusted such that the parsley leaf extract is contained in a ratio of 1 ppb to 500 ppm in terms of solid matter in 100% of the food or drink.

The parsley leaf extract in the present invention may be used alone or may be used in combination with an optional flavor deterioration inhibitor. In the case of using two or more kinds of flavor deterioration inhibitors in combination, the mixing ratio is not particularly limited, and the added amount of the mixed inhibitor varies depending on the purity of the components of the inhibitor to be used or the kind of the product to which the inhibitor is added, but the preparation is preferably adjusted such that the parsley leaf extract is contained in a ratio of 1 ppm to 50% in terms of solid matter in 100% of the flavor deterioration inhibitor.

In addition, in the case where the target product is a food or drink, the preparation is preferably adjusted such that the parsley leaf extract is contained in a ratio of 1 ppb to 500 ppm in terms of solid matter in 100% of the food or drink. The food or drink to which the flavor deterioration inhibitor of the present invention can be added is not particularly limited but is preferably a food or drink having a citrus flavor or a milk-containing food or drink, because the flavor deterioration inhibitor of the present invention is effective particularly in inhibiting the deterioration of a citrus flavor or a milk flavor. Examples of the food or drink having a citrus flavor include a carbonated drink, a fruit juice drink, a sports drink, an alcoholic drink, a tea/coffee drink, a soy drink, a functional drink, a soup, a frozen dessert, a candy/dessert, a chewing gum, confectionery such as baked goods, and a seasoning such as miso, soy sauce, sauce, baste and dressing. Examples of the milk-containing food or drink include a tea/coffee drink, a cocoa drink, a fermented milk, a milk-containing acidic drink, the above-described confectionery, yogurt, jelly, mousse, a frozen dessert such as ice cream, sherbet, pudding and cream, and butter, margarine, cheese or dairy·oil/fat products containing same.

## Examples

The present invention is described in detail below by referring to Examples, but the present invention is not limited to these Examples.

In Examples, mass% and ppm by mass are sometimes referred to as "%" and "ppm", respectively.

### (Extraction Example)

To 14 kg of parsley leaves that were washed using ethyl acetate and dried, 280 kg of 80% hydrous ethanol was added, and extraction was performed by stirring at 75°C for 2 hours. After solid-liquid separation, insoluble matters were removed by filtration, and the filtrate was left to stand still overnight at 4°C and then purified by adding activated carbon and silicon dioxide to obtain 234.5 kg of an extract. A part of the obtained extract was dispensed and subjected to vacuum concentration and freeze drying, and the solid weight in the extract was measured and found to be 1.57%.

Using the obtained parsley leaf extract, the inhibition activity against flavor deterioration was evaluated in Experimental Examples 1 to 3 below.

### (Experimental Example 1)

6.41 g of citric acid, 3.59 g of trisodium citrate, and 400 g of a sugar solution having a sugar content (Brix value) of 75 were mixed with 4,590 g of tap water to prepare 5,000 g of a saccharic acid solution having a pH of 3.5 and a sugar content (Brix value) of 6. Subsequently, a transparent PET bottle was filled with 350 g of the saccharic acid solution and after 100 ppm of citral and each flavor deterioration inhibitor in an amount to account for 50 ppm were added thereto, the resulting solution was subjected to a deterioration test. In addition, a citral-containing saccharic acid solution prepared by not adding a flavor deterioration inhibitor in the formulation above was similarly irradiated with a fluorescent lamp (+Light). A citral-containing saccharic acid solution to which a flavor deterioration inhibitor was not added and which was not irradiated with a fluorescent lamp was used as the control (-Light).

### (Light Deterioration Test)

A product to be tested that was prepared by filling a transparent PET bottle with a test specimen was irradiated with light by using a light stability tester ("LH30-14M", manufactured by Nagano Science Co., Ltd.). As for the irradiation conditions, irradiation with a white fluorescent lamp (illuminance: 10,000 lux) was performed at a temperature of 25°C

for 168 hours in a static state.

The citral-containing saccharic acid solution which has been subjected to the deterioration test above was concentrated by a solid phase extraction method. The solid phase used was conditioned with 500 mg/3 ml of "InertSep C18-B" manufactured by GL Science Inc. by flowing 2.25 ml of a pentane:ether (=1:1) solution, 2.25 ml of methanol and 4.5 ml of distilled water before sample loading. Subsequently, 100 ml of a solution under deterioration test was passed therethrough, followed by washing with 4.5 ml of distilled water, drying for 30 minutes, and then, allowing to elute with 4.5 ml of a pentane:ether (=1:1) solution. The eluate was dehydrated by adding a small amount of anhydrous sodium sulfate, and the solvent was removed by distillation in a nitrogen atmosphere. After adding 10 μl of 5-nonanone as the internal standard substance, the solution was made up to exactly 1 ml with ethanol by using a measuring flask. The thus-prepared sample was measured by gas chromatography, the citral residual amount and the amount of byproducts were analyzed.

(Measurement Conditions)

Apparatus: "6890N", manufactured by Agilent Technologies, Inc.
Column: "BC-WAX 0.25 mmx30 m, I.D. = 0.25 μm", manufactured by GL Science Inc.
Carrier gas: helium
Temperature conditions: from 60 to 230°C (temperature rise rate: 4°C/min)
Detector: FID (250°C)

From the measurement conditions above, the citral residual ratio (%) was calculated according to the following formula.

$$\text{Citral residual ratio (\%)} = (A/B) \times 100$$

A: the citral content in each sample after light irradiation,
B: the citral content in the sample not irradiated with light (control).

The results are shown in Table 1 and FIG. 1.

[Table 1]

| No. | Conditions | Citral Residual Ratio (%) |
|---|---|---|
| Comparative Example 1-1 | +Light | 67.3 |
| Comparative Example 1-2 | parsley, root region | 67.6 |
| Comparative Example 1-3 | parsley, stem region | 68.0 |
| Comparative Example 1-4 | αGRutinP | 73.2 |
| Comparative Example 1-5 | apigenin | 75.9 |
| Comparative Example 1-6 | apigenin (total P.P.) | 79.8 |
| Example 1-1 | parsley, leaf region | 91.4 |
| Control | -Light | 100.0 |

The symbols in Table 1 and FIG. 1 have the following meanings.

+Light: Irradiated with a fluorescent lamp (no addition of deterioration inhibitor)
parsley, root region: An extract obtained by the same operation by replacing leaf with root in the deterioration inhibitor of the present invention obtained in Extraction Example
parsley, stem region: An extract obtained by the same operation by replacing leaf with stem in the deterioration inhibitor of the present invention obtained in Extraction Example
αGRutinP: α-Glucosylrutin (produced by Toyo Sugar Refining Co., Ltd.)
apigenin: A deterioration inhibitor prepared by measuring the apigenin content in the deterioration inhibitor of the

present invention obtained in Extraction Example and regulating the concentration with an apigenin reagent produced by Cayman Chemical
apigenin (total P.P.): A deterioration inhibitor prepared by measuring the total polyphenol concentration in the deterioration inhibitor of the present invention obtained in Extraction Example and regulating the concentration with an apigenin reagent produced by Cayman Chemical
parsley, leaf region: The deterioration inhibitor of the present invention obtained in Extraction Example
-Light: Not irradiated with a fluorescent lamp (no addition of deterioration inhibitor)

The sample where a parsley leaf extract was added (Example 1-1) exhibited most prominent inhibition against decrease of citral due to light irradiation, compared with samples where a parsley root or stem region extract adjusted to the same concentration, αGRutinP, an apigenin reagent, or an apigenin reagent in an amount adjusted to match the polyphenol amount in the parsley leaf extract was added (Comparative Examples 1-1 to 1-6).
The dependency of the amount of the parsley leaf extract on the citral residual amount was evaluated.

(Experimental Example 2)

To a saccharic acid solution prepared under the same conditions as in Experimental Example 1, 100 ppm of citral and a parsley leaf extract in an amount to afford a solid content concentration of 0 ppm, 77 ppm or 314 ppm, respectively, were added, and after filling a transparent PET bottle with the resulting solution in the same manner as in the conditions of Experimental Example 1, a light deterioration test was performed.
The citral residual ratio (times) was calculated according to the following formula.

$$\text{Citral residual ratio (times)} = (C/D)$$

C: the citral content in each sample after light irradiation,
D: the citral content in the sample of Comparative Example 2-1 after light irradiation.

The results of dependency on the amount of the deterioration inhibitor added, when the citral residual amount in Comparative Example 2-1 is assumed to be 1.00, are shown in Table 2 and FIG. 2.

[Table 2]

| | Comparative Example 2-1 | Example 2-1 | Example 2-2 |
|---|---|---|---|
| Amount of parsley leaf extract added | 0 (Blank) | 77 ppm | 314 ppm |
| Citral residual amount (times) | 1.00 | 1.41 | 1.73 |

As shown in Table 2 and FIG. 2, when the parsley leaf extract was added, as the amount added is increased, the citral residual amount is larger, as compared with the case of addition of no extract. Thus, the effect of inhibiting citral deterioration due to light irradiation was exhibited dose, and the amount added was involved in the enhancement of the residual amount.
The dependency of the amount of parsley leaf extract on the byproduct inhibition was evaluated.

(Experimental Example 3)

As shown below, citral is known to decompose in the presence of light and produce photocitral A and photocitral B.
The amount of these citral-derived deterioration products was analyzed by gas chromatography, and the citral-derived light deterioration product production ratio (%) was calculated according to the following formula. As the test specimen, the specimen prepared in Experimental Example 2 was used.

$$\text{Citral-derived light deterioration product production ratio (\%)} = (E/F)\times 100$$

E: the citral-derived light deterioration product content in the sample after light irradiation,
F: the citral-derived light deterioration product content in the sample (comparative drink) after light irradiation.

[Chem. 1]

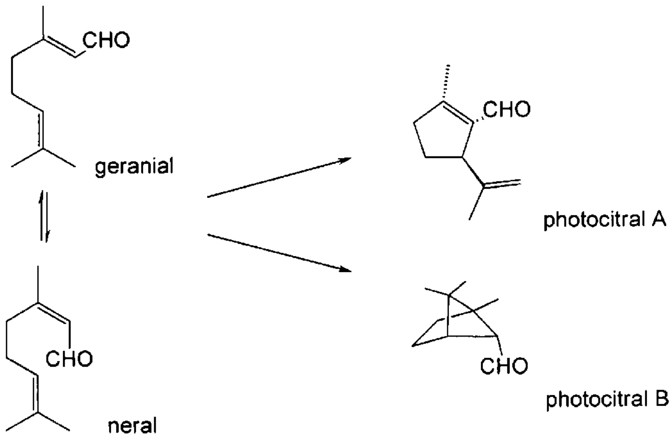
geranial
neral
photocitral A
photocitral B

The evaluation and measurement results of dependency on the amount of the deterioration inhibitor added, when the citral-derived light deterioration production production ratio in Comparative Example 3-1 is assumed to be 100%, are shown in Table 3 and FIG. 3.

[Table 3]

| | Comparative Example 3-1 | Example 3-1 | Example 3-2 |
|---|---|---|---|
| Amount of parsley leaf extract added | 0 (Blank) | 77 ppm | 314 ppm |
| Production ratio of photocitral A (%) | 100 | 33 | 12 |
| Production ratio of photocitral B (%) | 100 | 26 | 6 |

As seen in Table 3 and FIG. 3, as compared with the case of addition of no extract, when the parsley leaf extract was added, production of photocitral A and photocitral B, which are citral-derived light deterioration products, is strongly inhibited, and the effect of inhibiting light deterioration of citral was confirmed in an added amount-dependent manner.

(Evaluation Example 1)

(Milk)

A glass bottle was filled with 100 g of milk in which various additives were added to account for 0.05%, and placed in a light stability tester, and after irradiation with a fluorescent lamp (5,000 lux, 5°C, 7 hours), a sensory evaluation was performed by 10 trained panelists selected. A milk (control) to which a flavor deterioration inhibitor and additives were not added and which was not irradiated with a fluorescent lamp was prepared, and the degree of flavor deterioration was relatively evaluated by scores ranging from score 7 where the flavor is not changed in comparison with the control, to score 1 where the flavor is significantly deteriorated, by 1 point. The results are shown in Table 4 and FIG. 4. The score for evaluation in Table 4 is an average score of each of the panelists rating the sample according to the following criteria. Evaluation Examples 2 to 5 below were performed by the same evaluation method.

Sensory Evaluation Criteria:

7: The milk has the same flavor as the control.
1: The flavor is significantly deteriorated.

[Table 4]

| (Milk) | | | | |
|---|---|---|---|---|
| Additive | Parsley Leaf Extract (PSL) | αGRutinP | Super-Emulsion TSM-09 | None (+Light) |
| Texture | 4.8 | 3.8 | 3.0 | 1.6 |
| Flavor on palate | 4.2 | 3.2 | 2.6 | 1.0 |
| Smoothness of drinking | 4.8 | 3.6 | 2.8 | 1.8 |
| Sweetness | 5.6 | 5.4 | 4.8 | 4.4 |
| Fresh feeling | 4.6 | 3.2 | 2.4 | 1.0 |
| Richness | 5.2 | 5.0 | 3.4 | 3.2 |
| Aftertaste | 4.8 | 4.0 | 2.8 | 2.4 |
| Fatty feeling | 5.2 | 4.8 | 4.2 | 3.0 |
| Overall judgment | 4.2 | 3.2 | 1.8 | 1.0 |

Symbols in Table 4 and FIG. 4 have the following meanings (the same in the following Evaluation Examples).

Parsley leaf extract: The deterioration inhibitor of the present invention obtained in Extraction Example
αGRutinP: α-Glucosylrutin (produced by Toyo Sugar Refining Co., Ltd.)
Super-Emulsion TSM-09: Tocopherol and tea extract (produced by Taiyo Kagaku Co., Ltd.)

(Evaluation Example 2)

(Soft Ice-Cream Mix)

A transparent plastic container was filled with 100 g of soft ice-cream mix in which various additives were added to account for 0.05%, and after irradiation with a fluorescent lamp (5,000 lux, 5°C, 24 hours), the same evaluation as in Evaluation Example 1 was performed. The results are shown in Table 5 and FIG. 5.

[Table 5]

| (Soft Ice-Cream) | | | | |
|---|---|---|---|---|
| Additive | Parsley Leaf Extract (PSL) | αGRutinP | Super-Emulsion TSM-09 | None (+Light) |
| Texture | 5.2 | 4.8 | 4.0 | 2.7 |
| Flavor on palate | 4.2 | 3.7 | 2.8 | 1.2 |
| Sweetness | 6.3 | 6.2 | 5.5 | 5.5 |
| Fresh feeling | 4.8 | 4.2 | 3.3 | 1.5 |
| Richness | 5.8 | 5.8 | 5.0 | 4.8 |
| Aftertaste | 4.7 | 4.2 | 2.8 | 2.2 |
| Fatty feeling | 6.0 | 5.8 | 5.0 | 4.7 |
| Overall judgment | 4.3 | 3.8 | 2.5 | 1.2 |

(Evaluation Example 3)

(Milk Pudding)

A milk pudding base was prepared by putting and warming 600 g of milk and 100 g of sugar, adding 15 g of powder gelatin dissolved in 10 g of water, and homogenizing the mixture, and various additives were added to the base so as to account for 0.05%. A transparent plastic container was filled with 100 g of the milk pudding base having added thereto various additives, and the base was hardened by cooling overnight in a refrigerator and then irradiated with a

fluorescent lamp (5,000 lux, 5°C, 7 hours). Thereafter, the same evaluation as in Evaluation Example 1 was performed, and the results are shown in Table 6 below and FIG. 6.

[Table 6]

| (Milk Pudding) | | | | |
|---|---|---|---|---|
| Additive | Parsley Leaf Extract (PSL) | αGRutinP | Super-Emulsion TSM-09 | None (+Light) |
| Texture | 6.0 | 5.4 | 5.4 | 4.8 |
| Flavor on palate | 5.2 | 4.6 | 3.8 | 2.4 |
| Sweetness | 5.8 | 5.6 | 5.2 | 4.0 |
| Fresh feeling | 5.4 | 4.4 | 3.6 | 2.4 |
| Richness | 5.4 | 5.0 | 4.6 | 4.4 |
| Aftertaste | 5.8 | 5.0 | 4.2 | 3.2 |
| Fatty feeling | 6.0 | 5.0 | 5.0 | 4.8 |
| Overall judgment | 5.0 | 4.6 | 3.6 | 2.8 |

(Evaluation Example 4)

(Whipped Cream)

After adding 15 g of sugar to 200 g of cream, various additives were added so as to account for 0.05%, and the cream was whipped rapidly under ice cooling. The whipped cream was transferred to a squeezer and after filling a transparent plastic container with an appropriate amount, the cream was irradiated with a fluorescent lamp (5,000 lux, 5°C, 24 hours). Thereafter, the same evaluation as in Evaluation Example 1 was performed, and the results are shown in Table 7 below and FIG. 7.

[Table 7]

| (Whipped Cream) | | | | |
|---|---|---|---|---|
| Additive | Parsley Leaf Extract (PSL) | αGRutinP | Super-Emulsion TSM-09 | None (+Light) |
| Texture | 6.8 | 6.5 | 6.3 | 6.0 |
| Flavor on palate | 5.8 | 4.5 | 3.3 | 3.3 |
| Sweetness | 6.0 | 5.3 | 4.5 | 4.0 |
| Fresh feeling | 6.0 | 5.5 | 4.0 | 3.8 |
| Richness | 6.5 | 5.8 | 5.3 | 5.5 |
| Aftertaste | 5.8 | 4.5 | 3.8 | 3.5 |
| Fatty feeling | 6.3 | 5.8 | 5.3 | 4.8 |
| Overall judgment | 5.8 | 4.8 | 4.3 | 3.0 |

(Evaluation 5)

(Yogurt)

A transparent plastic container was filled with 95 g of yogurt in which various additives were added so as to account for 0.05%, and the yogurt was irradiated with a fluorescent lamp (1,000 lux, 5°C, 10 days). Thereafter, the same evaluation as in Evaluation Example 1 was performed, and the results are shown in Table 8 below and FIG. 8.

[Table 8]

| (Yogurt) | | | | |
|---|---|---|---|---|
| Additive | Parsley Leaf Extract (PSL) | αGRutinP | Super-Emulsion TSM-09 | None (+Light) |
| Texture | 6.0 | 5.4 | 5.0 | 3.8 |
| Flavor on palate | 5.2 | 4.6 | 3.6 | 2.0 |
| Sweetness | 5.8 | 4.8 | 4.6 | 3.6 |
| Fresh feeling | 5.4 | 4.4 | 3.4 | 2.0 |
| Richness | 5.4 | 5.0 | 4.0 | 3.4 |
| Aftertaste | 5.8 | 5.0 | 3.4 | 3.2 |
| Fatty feeling | 6.0 | 5.0 | 3.8 | 3.0 |
| Overall judgment | 5.6 | 4.6 | 3.6 | 2.4 |

(Examples 4 to 9)

Preparation of Food or Drink:

Various foods or drinks were prepared according to the formulation shown in Tables 9 to 14 below by adding, as the flavor deterioration inhibitor, 0.001% of various additives such as deterioration inhibitor of the present invention. These foods or drinks were subjected to irradiation with a fluorescent lamp (5,000 lux, 5°C, 7 days) or heat deterioration (50°C, 10 days, light-shielded state), and thereafter, a sensory evaluation was performed by 10 trained panelists selected. As for the light deterioration test, a food or drink (control) to which a flavor deterioration inhibitor was not added and which was not irradiated with a fluorescent lamp was prepared, and as for the heat deterioration test, a food or drink (control) to which a flavor deterioration inhibitor was not added and which was stored in a static state under refrigeration was prepared. The degree of flavor deterioration was relatively evaluated by scores ranging from score 7 where the flavor is not changed in comparison with the control, to score 1 where the flavor is significantly deteriorated, by 1 point. The results are shown in Table 15.

[Table 9]

| Sports Drink | % |
|---|---|
| Granulated sugar | 3.10 |
| Fructose glucose liquid sugar | 1.57 |
| Citric acid | 0.10 |
| Vitamin C | 0.08 |
| Calcium Lactate | 0.07 |
| Sodium citrate | 0.03 |
| Sodium chloride | 0.03 |
| Potassium chloride | 0.02 |
| Sodium L-glutamate | 0.003 |
| Niacin | 0.0013 |
| Sodium panthenoate | 0.0007 |
| Vitamin B6 | 0.00022 |
| Vitamin B 12 | 0.0000006 |
| Grapefruit flavor | 0.10 |
| Water | balance |

[Table 10]

| Lemon juice-containing soft drink | % |
|---|---|
| Fructose glucose liquid sugar | 8.00 |
| 6.5-Fold concentrated lemon juice | 0.80 |
| Citric acid | 0.13 |
| Trisodium citrate | 0.07 |
| Lemon flavor | 0.10 |
| Water | balance |

[Table 11]

| Apple juice-containing soft drink | % |
|---|---|
| Fructose glucose liquid sugar | 8.00 |
| 5-Fold concentrated lemon juice | 2.00 |
| Citric acid | 0.13 |
| Trisodium citrate | 0.07 |
| Apple flavor | 0.10 |
| Water | balance |

[Table 12]

| Fruit juice-free orange-flavored carbonated drink | % |
|---|---|
| Fructose glucose liquid sugar | 13.00 |
| Citric acid | 0.10 |
| Orange flavor | 0.10 |
| Water | balance |

[Table 13]

| Lemon tea | % |
|---|---|
| Fructose glucose liquid sugar | 6.00 |
| Black tea leaf | 1.00 |
| 5-Fold concentrated lemon juice | 0.20 |
| Vitamin C | 0.01 |
| Lemon flavor | 0.10 |
| Water | balance |

[Table 14]

| Milk tea | % |
|---|---|
| Milk | 20.00 |
| Granulated sugar | 3.00 |

(continued)

| Milk tea | % |
|---|---|
| Black tea leaf | 1.00 |
| Milk flavor | 0.10 |
| Emulsifier | 0.03 |
| Water | balance |

[Table 15]

| Model Drink | Additive | Light Deterioration Test | Heat Deterioration Test |
|---|---|---|---|
| Sports drink | α-glucosylrutin | 3.4 | 1.6 |
| | TSM-09 | 1.6 | 2.8 |
| | parsley leaf extract | 6.2 | 5.6 |
| Lemon juice-containing soft drink | α-glucosylrutin | 3.2 | 1.8 |
| | TSM-09 | 1.4 | 2.8 |
| | parsley leaf extract | 5.8 | 6.2 |
| Apple juice-containing soft drink | α-glucosylrutin | 2.8 | 2.0 |
| | TSM-09 | 2.2 | 2.6 |
| | parsley leaf extract | 5.6 | 5.4 |
| Fruit juice-free orange-flavored carbonated drink | α-glucosylrutin | 3.0 | 2.4 |
| | TSM-09 | 1.8 | 2.4 |
| | parsley leaf extract | 5.4 | 5.8 |
| Lemon tea | α-glucosylrutin | 2.6 | 1.8 |
| | TSM-09 | 1.8 | 2.4 |
| | parsley leaf extract | 5.8 | 5.6 |
| Milk tea | α-glucosylrutin | 2.8 | 1.6 |
| | TSM-09 | 2.0 | 3.2 |
| | parsley leaf extract | 6.2 | 6.0 |

The symbols in Table 15 have the following meanings.

parsley leaf extract: The deterioration inhibitor of the present invention obtained in Extraction Example
α-glucosylrutin: Produced by Toyo Sugar Refining Co., Ltd.
TSM-09: Tocopherol and tea extract (produced by Taiyo Kagaku Co., Ltd.)

(Example 7)

Preparation of Gummi Candy

After 400 g of starch syrup and 400 g of granulated sugar were heated/dissolved and cooled, a gelatin solution

prepared by mixing 70 g of gelatin and 130 g of water was added thereto and mixed therewith. 3 g of lemon flavor, 30 g of an aqueous 50% citric acid solution, and 0.3 g of an ethanol solution containing 2% of various flavor deterioration inhibitors were added thereto, followed by stirring. The resulting solution was cast into a mold and dried overnight. These gummy candies were put in a light stability tester and after irradiation with a fluorescent lamp (10,000 lux, 25°C, 100 hours), a sensory evaluation was performed by 10 trained panelists selected. A gummy candy (control) to which a flavor deterioration inhibitor was not added and which was not irradiated with a fluorescent lamp was prepared. The results of change in color tone and the results when the degree of flavor deterioration was relatively evaluated by rating the control as score 5 are shown in Table 16. The score for evaluation in Table 16 is an average score of each of the panelists rating the sample according to the following criteria.

Color tone: Relatively evaluated by scores ranging from score 5 where the color is not changed in comparison with the control, to score 1 where the color is completely faded, by 1 point.
Sensory evaluation: Relatively evaluated by scores ranging from score 5 where the flavor is not changed in comparison with the control, to score 1 where the flavor is significantly deteriorated, by 1 point.

[Table 16]

| | | Color Tone | Sensory Evaluation | Comment |
|---|---|---|---|---|
| Control | light-shielded | 5 | 5 | - |
| Control | irradiated with light | 1 | 1 | Flavor is lost from top, overall weak flavor |
| V.E. | irradiated with light | 2 | 2 | bad smell originated from material is felt, deterioration is slightly inhibited but heavy impression |
| Parsley leaf extract | irradiated with light | 4 | 4 | no off-taste, no off-flavor, and deterioration is inhibited |

The symbols in Table 16 have the following meanings.

Parsley leaf extract: The deterioration inhibitor of the present invention obtained in Extraction Example
V.E.: Riken E-Oil Super 80 (produced by Riken Vitamin Co., Ltd.)

While the invention has been described in detail and with reference to specific embodiments thereof, it will be apparent to one skilled in the art that various changes and modifications can be made therein without departing from the spirit and scope of the invention. This application is based on Japanese Patent Application No. 2013-052133 filed on March 14, 2013, the contents of which are incorporated herein by way of reference.

Industrial Applicability

As described in the foregoing pages, the flavor deterioration inhibitor of the present invention can be added to various foods or drinks and utilized for inhibiting flavor deterioration due to light of those products.

## Claims

1. Use of a solvent extract of a parsley leaf as a flavor deterioration inhibitor for inhibiting flavor deterioration due to light.

2. The use according to claim 1, wherein the solvent extract is obtained by extraction with water, an organic solvent or a mixture thereof.

3. A method for inhibiting flavor deterioration of a food or drink due to light, comprising adding a flavor deterioration inhibitor in an amount of from 1 ppb by mass to 500 ppm by mass in terms of solid weight to a food or drink, the flavor deterioration inhibitor comprising a solvent extract of a parsley leaf.

4. The method for inhibiting flavor deterioration of a food or drink according to claim 3, wherein the solvent extract is

obtained by extraction with water, an organic solvent or a mixture thereof.

5. A method for inhibiting flavor deterioration of a flavor due to light comprising adding a flavor deterioration inhibitor in an amount of from 1 ppm by mass to 50 mass% in terms of solid weight to a flavor, the flavor deterioration inhibitor comprising a solvent extract of a parsley leaf.

6. The method for inhibiting flavor deterioration of a flavor according to claim 5, wherein the solvent extract is obtained by extraction with water, an organic solvent or a mixture thereof.

## Patentansprüche

1. Verwendung eines Lösungsmittelextrakts eines Petersilienblatts als Aromaverschlechterungsinhibitor zum Inhibieren einer Aromaverschlechterung aufgrund von Licht.

2. Verwendung nach Anspruch 1, wobei der Lösungsmittelextrakt durch Extraktion mit Wasser, einem organischen Lösungsmittel oder einem Gemisch davon erhalten wird.

3. Verfahren zum Inhibieren der Aromaverschlechterung eines Nahrungsmittels oder Getränks aufgrund von Licht, umfassend Zugeben eines Aromaverschlechterungsinhibitors in einer Menge von 1 ppb bezogen auf Masse bis 500 ppm bezogen auf Masse hinsichtlich des Feststoffgewichts zu einem Nahrungsmittel oder Getränk, wobei der Aromaverschlechterungsinhibitor einen Lösungsmittelextrakt eines Petersilienblatts umfasst.

4. Verfahren zum Inhibieren der Aromaverschlechterung eines Nahrungsmittels oder Getränks nach Anspruch 3, wobei der Lösungsmittelextrakt durch Extraktion mit Wasser, einem organischen Lösungsmittel oder einem Gemisch davon erhalten wird.

5. Verfahren zum Inhibieren der Aromaverschlechterung eines Aromas aufgrund von Licht, umfassend Zugeben eines Aromaverschlechterungsinhibitors in einer Menge von 1 ppm bezogen auf Masse bis 50 Massen-% hinsichtlich des Feststoffgewichts zu einem Aroma, wobei der Aromaverschlechterungsinhibitor einen Lösungsmittelextrakt eines Petersilienblatts umfasst.

6. Verfahren zum Inhibieren der Aromaverschlechterung eines Aromas nach Anspruch 5, wobei der Lösungsmittelextrakt durch Extraktion mit Wasser, einem organischen Lösungsmittel oder einem Gemisch davon erhalten wird.

## Revendications

1. Utilisation d'un extrait par solvant d'une feuille de persil en tant qu'inhibiteur de détérioration de saveur afin d'inhiber la détérioration de saveur due à la lumière.

2. Utilisation selon la revendication 1, dans laquelle l'extrait par solvant est obtenu par extraction avec de l'eau, un solvant organique ou un mélange de ces derniers.

3. Procédé d'inhibition de détérioration de saveur d'un aliment ou boisson due à la lumière, comprenant l'ajout d'un inhibiteur de détérioration de saveur en une quantité de 1 ppb en masse à 500 ppm en masse en termes de poids de solide à un aliment ou une boisson, l'inhibiteur de détérioration de saveur comprenant un extrait par solvant d'une feuille de persil.

4. Procédé d'inhibition de détérioration de saveur d'un aliment ou boisson selon la revendication 3, dans lequel l'extrait par solvant est obtenu par extraction avec de l'eau, un solvant organique ou un mélange de ces derniers.

5. Procédé d'inhibition de détérioration de saveur d'une saveur due à la lumière comprenant l'ajout d'un inhibiteur de détérioration de saveur en une quantité de 1 ppm en masse à 50 % en masse en termes de poids de solide à une saveur, l'inhibiteur de détérioration de saveur comprenant un extrait par solvant d'une feuille de persil.

6. Procédé d'inhibition de détérioration de saveur d'une saveur selon la revendication 5, dans lequel l'extrait par solvant est obtenu par l'extraction avec de l'eau, un solvant organique ou un mélange de ces derniers.

FIG. 1

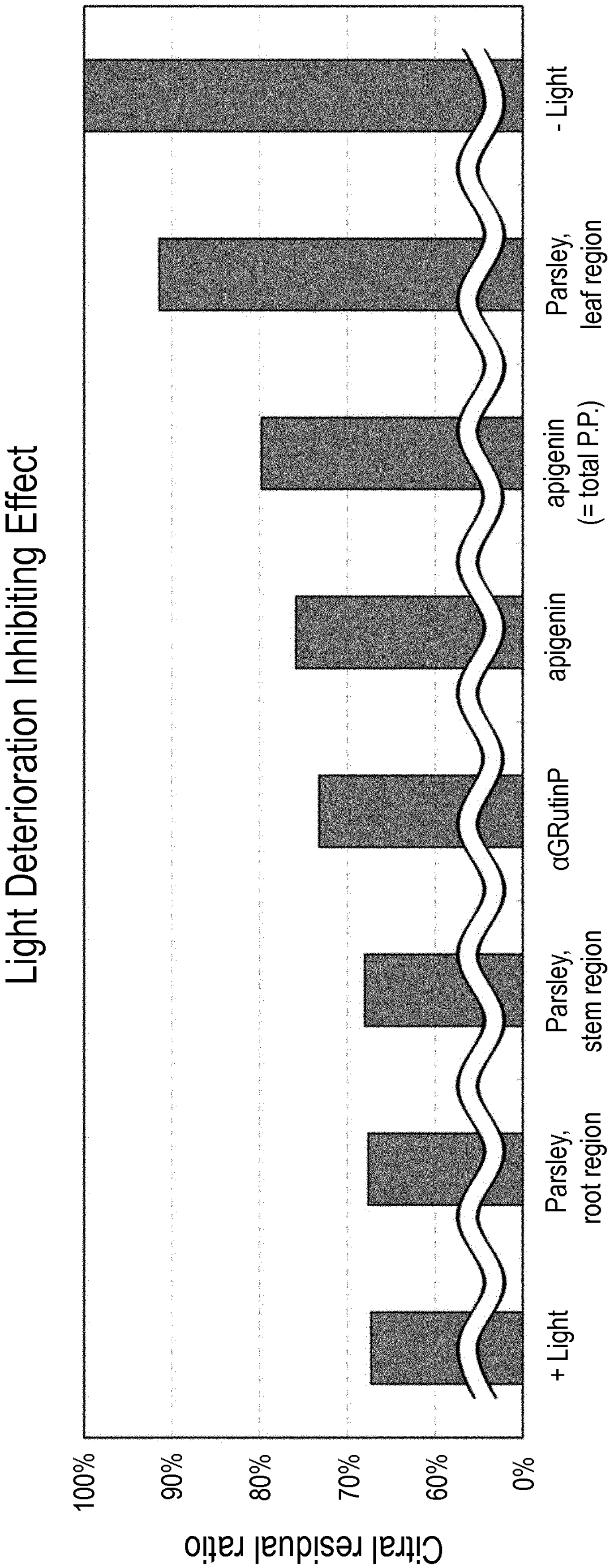

Light Deterioration Inhibiting Effect
Citral residual ratio
100%
90%
80%
70%
60%
0%
+ Light
Parsley, root region
Parsley, stem region
αGRutinP
apigenin
apigenin (= total P.P.)
Parsley, leaf region
- Light

FIG. 2

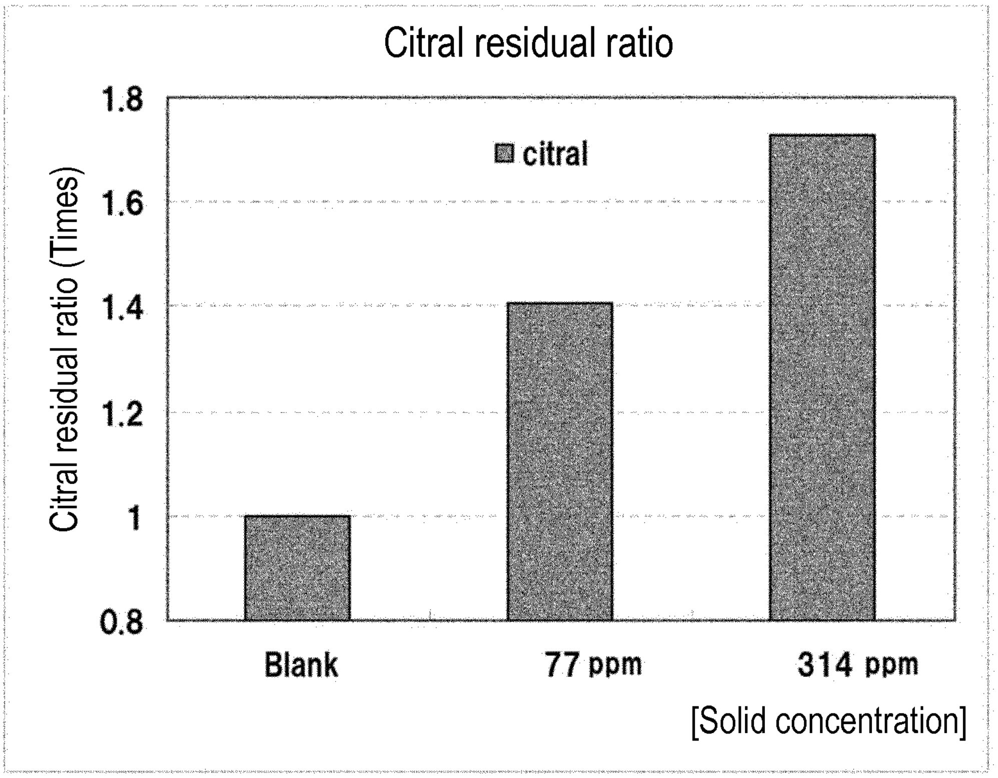
Citral residual ratio
citral
Citral residual ratio (Times)
1.8
1.6
1.4
1.2
1
0.8
Blank
77 ppm
314 ppm
[Solid concentration]

FIG. 3

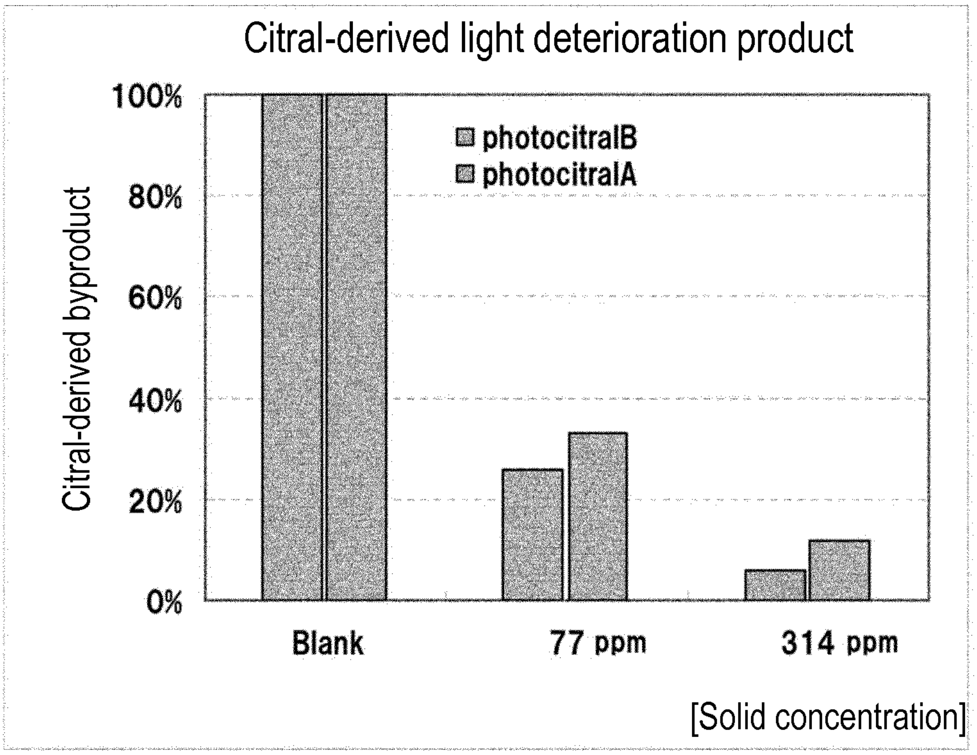
Citral-derived light deterioration product
100%
80%
60%
40%
20%
0%
Citral-derived byproduct
photocitralB
photocitralA
Blank
77 ppm
314 ppm
[Solid concentration]

FIG. 4

[Milk]

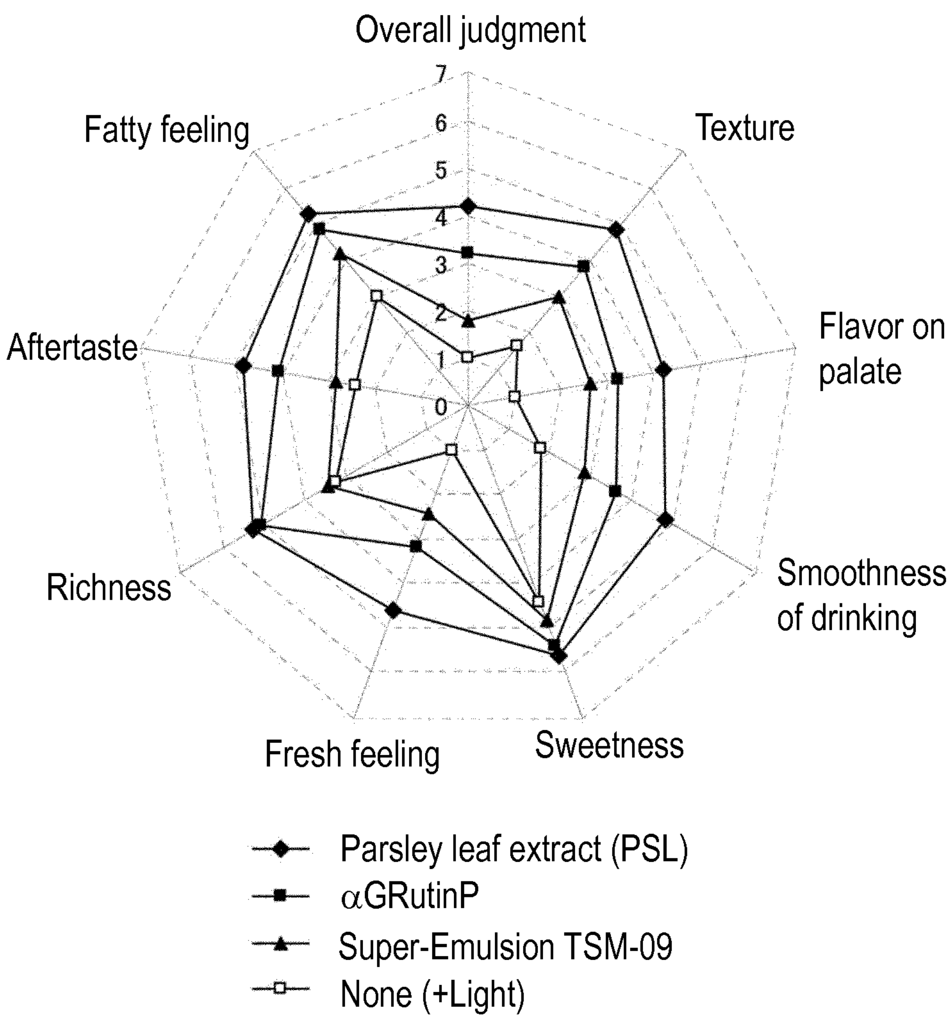
Overall judgment
Texture
Flavor on palate
Smoothness of drinking
Sweetness
Fresh feeling
Richness
Aftertaste
Fatty feeling
7
6
5
4
3
2
1
0
Parsley leaf extract (PSL)
αGRutinP
Super-Emulsion TSM-09
None (+Light)

FIG. 5

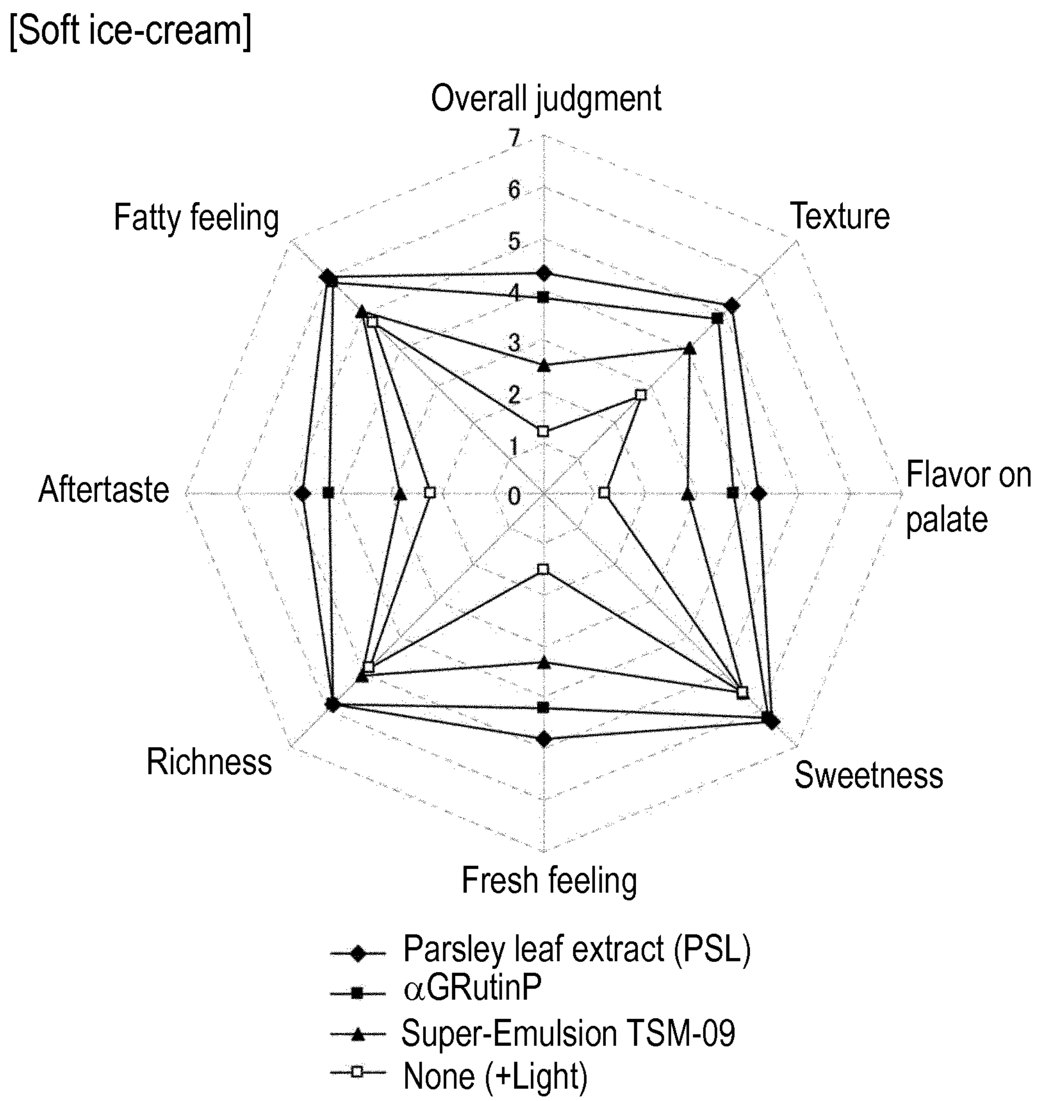
[Soft ice-cream]
Overall judgment
Texture
Flavor on palate
Sweetness
Fresh feeling
Richness
Aftertaste
Fatty feeling
0
1
2
3
4
5
6
7
Parsley leaf extract (PSL)
αGRutinP
Super-Emulsion TSM-09
None (+Light)

FIG. 6

[Milk pudding]

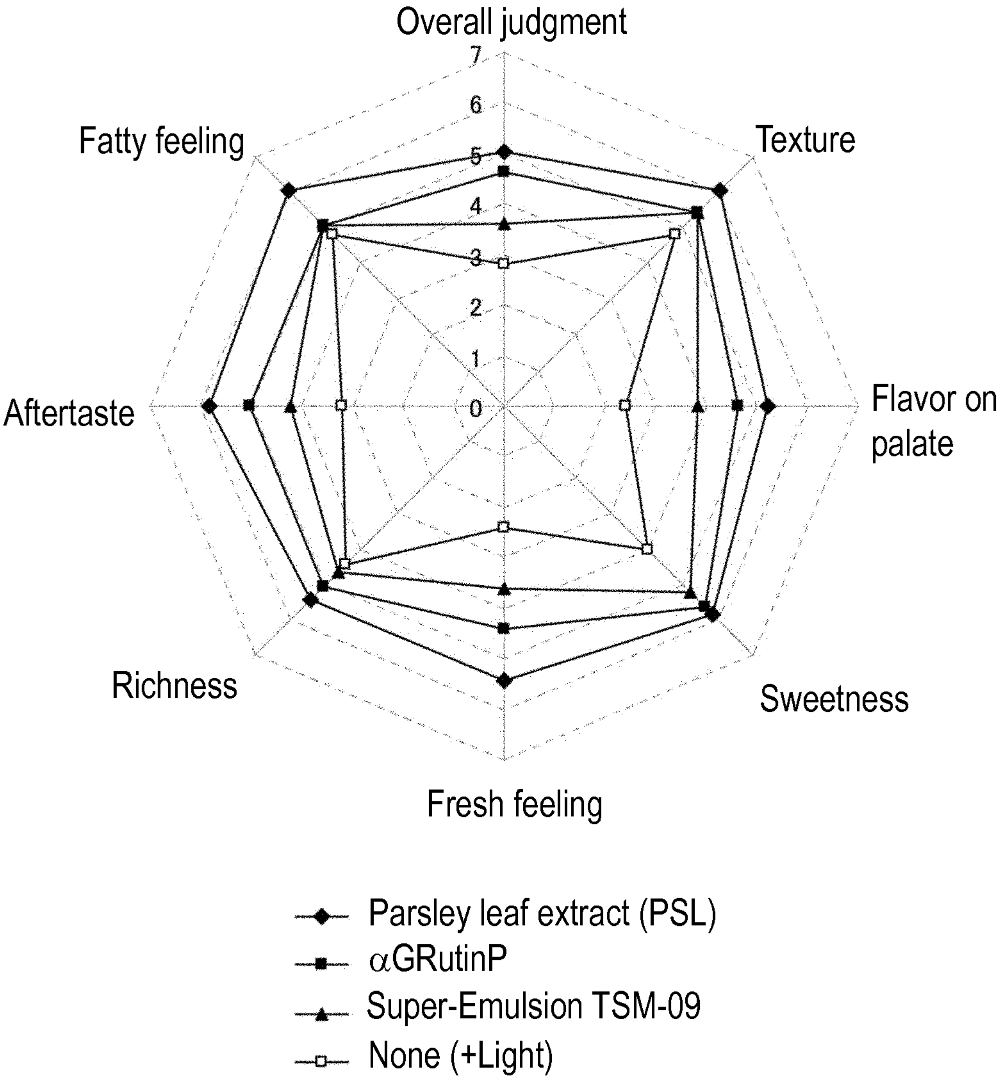

FIG. 7

[Whipped cream]

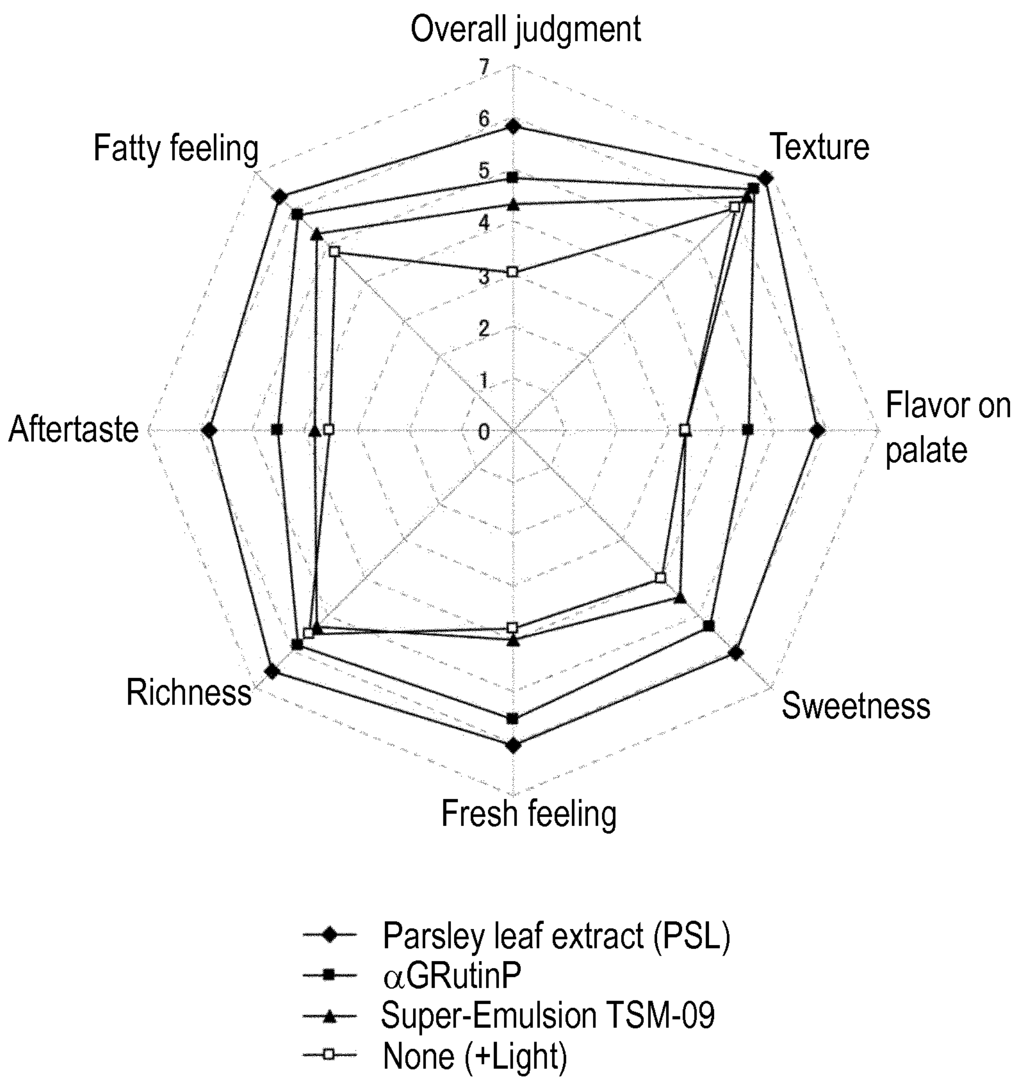

FIG. 8

[Yogurt]

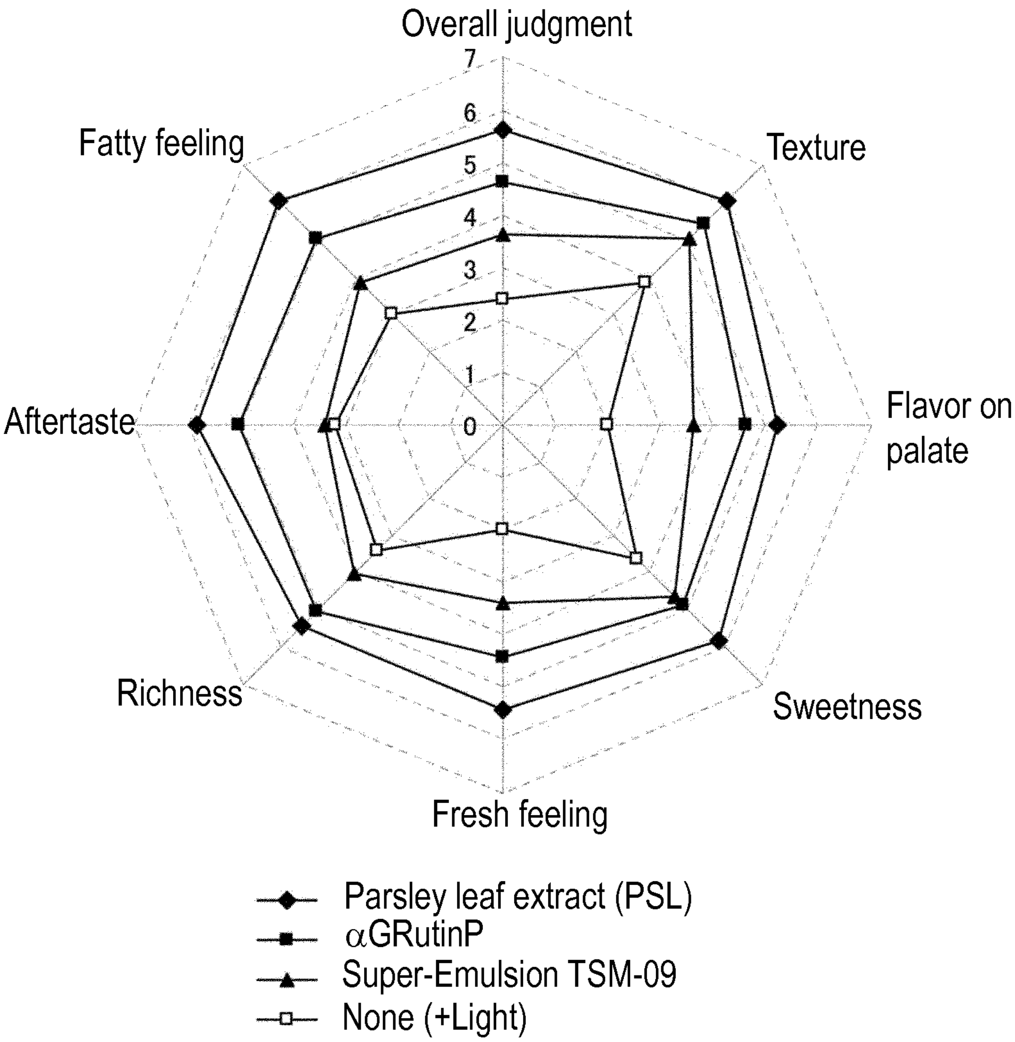
Overall judgment
Texture
Flavor on palate
Sweetness
Fresh feeling
Richness
Aftertaste
Fatty feeling
0
1
2
3
4
5
6
7
Parsley leaf extract (PSL)
αGRutinP
Super-Emulsion TSM-09
None (+Light)

FIG. 9

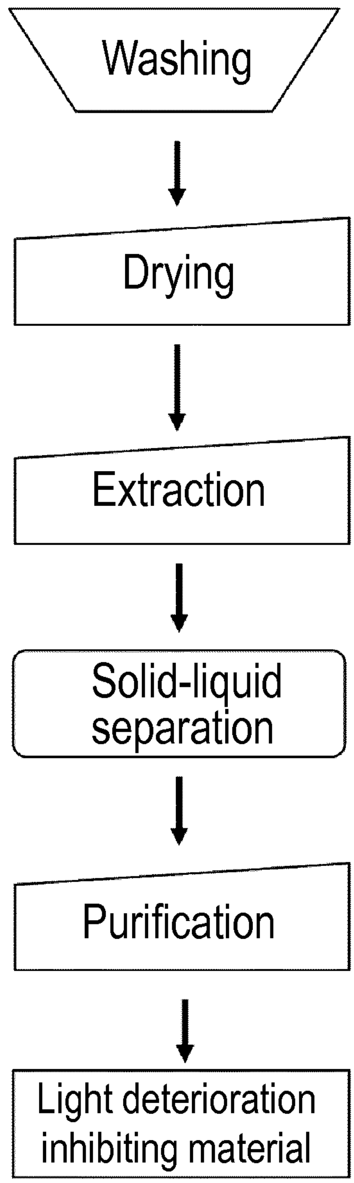
Washing
Drying
Extraction
Solid-liquid separation
Purification
Light deterioration inhibiting material

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001136931 A
- JP 3027293 A
- JP 2004166631 A
- JP 2013052133 A


### Non-patent literature cited in the description


- *British Journal of Nutrition,* 1999, vol. 81 (6), 447-455
- **BIN SHAN ; YIZHONG Z. CAI ; MEI SUN ; HAROLD CORKE.** *JOURNAL OF AGRICALTURAL AND FOOD CHEMISTRY,* 2005, vol. 53, 7749-7759